(19) Europäisches Patentamt
European Patent Office
Office européen des brevets

(11) Veröffentlichungsnummer: **0 040 742**
**B1**

(12) # EUROPÄISCHE PATENTSCHRIFT

(45) Veröffentlichungstag der Patentschrift: **13.07.83**

(51) Int. Cl.³: **C 07 C 69/743**, C 07 C 67/08, A 01 N 53/00

(21) Anmeldenummer: **81103581.5**

(22) Anmeldetag: **11.05.81**

(54) **3-(2-Chlor-3,3,4,4,4-pentafluor-1-butenyl)-2,2-dimethyl-cyclopropancarbonsäureester, Verfahren zu deren Herstellung und deren Verwendung in Schädlingsbekämpfungsmitteln.**

(30) Priorität: **22.05.80 DE 3019552**

(43) Veröffentlichungstag der Anmeldung: **02.12.81 Patentblatt 81/48**

(45) Bekanntmachung des Hinweises auf die Patenterteilung: **13.07.83 Patentblatt 83/28**

(84) Benannte Vertragsstaaten: **AT BE CH DE FR GB IT LI NL SE**

(56) Entgegenhaltungen: **EP A 0 008 340**

**Die Akte enthält technische Angaben, die nach dem Eingang der Anmeldung eingereicht wurden und die nicht in dieser Patentschrift enthalten sind.**

(73) Patentinhaber: **BAYER AG**
**Zentralbereich Patente, Marken und Lizenzen**
**D-5090 Leverkusen 1, Bayerwerk (DE)**

(72) Erfinder: **Fuchs, Rainer, Dr.**
**Roeberstrasse 8**
**D-5600 Wuppertal 1 (DE)**
Erfinder: **Naumann, Klaus, Dr.**
**Richard-Wagner-Strasse 9**
**D-5090 Leverkusen (DE)**
Erfinder: **Lantzsch, Reinhard, Dr.**
**Heymannstrasse 32**
**D-5090 Leverkusen (DE)**
Erfinder: **Hagemann, Hermann, Dr.**
**Kadinsky Strasse 52**
**D-5090 Leverkusen 1 (DE)**
Erfinder: **Hammann, Ingeborg, Dr.**
**Belfortstrasse 9**
**D-5000 Koeln (DE)**
Erfinder: **Homeyer, Bernhard, Dr.**
**Obere Strasse 28**
**D-5090 Leverkusen 3 (DE)**
Erfinder: **Behrenz, Wolfgang, Dr.**
**Untergruendemich 14**
**D-5063 Overath (DE)**
Erfinder: **Stendel, Wilhelm, Dr.**
**In den Birken 55**
**D-5600 Wuppertal 1 (DE)**

**EP 0 040 742 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

3-(2-Chlor-3,3,4,4,4-pentafluor-1-butenyl)-2,2-dimethyl-cyclopropancarbonsäureester, Verfahren zu deren Herstellung und deren Verwendung in Schädlingsbekämpfungsmitteln

Die Erfindung betrifft neue 3-(2-Chlor-3,3,4,4,4-pentafluor-1-butenyl)-2,2-dimethyl-cyclopropancarbonsäureester, ein Verfahren zu deren Herstellung sowie deren Verwendung in Schädlingsbekämpfungsmitteln, insbesondere in Insektiziden und Akariziden.

Es ist bekannt, daß bestimmte substituierte Cyclopropancarbonsäureester, wie z. B. 3-(2-Methyl-1-propenyl)-2,2-dimethyl-cyclopropancarbonsäure-(3-phenoxy-benzyl)-ester (Phenothrin) und 3-(2,2-Dichlorvinyl-2,2-dimethyl-cyclopropancarbonsäure-(3-phenoxy-benzyl)-ester (Permethrin) insektizide und akarizide Wirkung aufweisen. (vgl. GB-PS 1 243 858 und 1 413 491).

Die Wirkung dieser Verbindungen ist jedoch, insbesondere bei niedrigen Wirkstoffkonzentrationen und Aufwandmengen, nicht immer zufriedenstellend.

Es wurden nun neue 3-(2-Chlor-3,3,4,4,4-pentafluor-1-butenyl)-2,2-dimethyl-cyclopropancarbonsäureester der Formel I

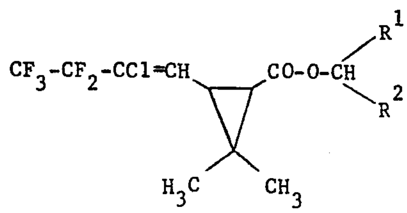

(I)

gefunden, in welcher

$R^1$ für Wasserstoff oder Cyano steht und

$R^2$ für einen durch Fluor und/oder Phenoxy substituierten Phenyl-Rest steht, mit der Maßgabe, daß der Rest $R^2$ insgesamt wenigstens einen Fluor-Substituenten enthält.

Die allgemeine Formel (I) schließt die verschiedenen möglichen Stereoisomeren und optisch aktiven Isomeren sowie deren Mischungen mit ein.

Man erhält die neuen Verbindungen der Formel (I), wenn man 3-(2-Chlor-3,3,4,4,4-pentafluor-1-butenyl)-2,2-dimethyl-cyclopropancarbonsäure der Formel II

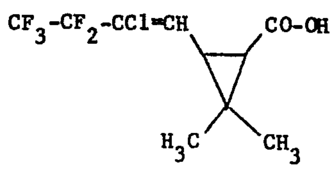

(II)

oder reaktionsfähige Derivate derselben, mit Benzylalkoholen der Formel III

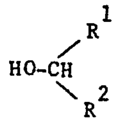

(III)

in welcher

$R^1$ und $R^2$ die oben angegebenen Bedeutungen haben, oder mit reaktionsfähigen Derivaten derselben, gegebenenfalls in Gegenwart von Säureakzeptoren und/oder Katalysatoren und gegebenenfalls unter Verwendung von Verdünnungsmitteln umsetzt.

Die neuen 3-(2-Chlor-3,3,4,4,4-pentafluor-1-butenyl)-2,2-dimethyl-cyclopropancarbonsäureester der Formel (I) zeichnen sich durch hohe insektizide und akarizide Wirksamkeit aus.

Überraschenderweise zeigen die erfindungsgemäßen Verbindungen der Formel (I) eine erheblich höhere insektizide und akarizide Wirkung als aus dem Stand der Technik bekannte Verbindungen analoger Konstitution und gleicher Wirkungsrichtung.

Gegenstand der Erfindung sind vorzugsweise Verbindungen der Formel (I), in welcher

$R^1$ für Wasserstoff oder Cyano steht und

$R^2$ für Pentafluorphenyl oder 4-Fluor-3-phenoxy-phenyl steht.

In einer bevorzugten Variante (a) des herstellungsverfahrens für die Verbindungen der Formel (I) wird 3-(2-Chlor-3,3,4,4,4-pentafluor-1-butenyl)-2,2-dimethyl-cyclopropancarbonsäure-chlorid der Formel IIa

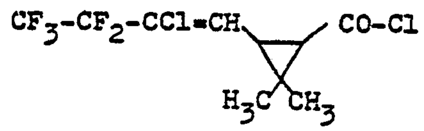

(IIa)

mit Benzylalkoholen der Formel III (oben) in Gegenwart von Säureakzeptoren und unter Verwendung von Verdünnungsmitteln umgesetzt.

In einer weiteren bevorzugten Verfahrensvariante (b), insbesondere zur herstellung von Verbindungen der Formel (I), in welcher $R^1$ für Cyano und $R^2$ für fluor-substituiertes Phenoxy-phenyl steht, wird das Säurechlorid der Formel IIa (oben) mit entsprechenden Phenoxy-benzaldehyden der Formel IV

$$OHC{-}R^2 \qquad \text{(IV)}$$

in welcher

$R^2$ für fluor-substituiertes Phenoxy-phenyl steht, und wenigstens der äquimolaren Menge eines Alkalicyanids (Natrium- oder Kalium-cyanid) in Gegenwart von Wasser und eines mit Wasser nicht mischbaren organischen Lösungsmittels und gegebenenfalls in Gegenwart eines Katalysators umgesetzt.

Als weitere reaktionsfähige Derivate der Carbonsäure der Formel (II) sind deren Niederalkylester zu nennen, welche mit Alkoholen der Formel (III) nach üblichen Methoden umgesetzt werden können.

Alkali-, Erdalkali- oder Ammoniumsalze der Carbonsäure (II) können mit Benzylhalogeniden, welche sich von den Benzylalkoholen der Formel (III) ableiten, ebenfalls zu Verbindungen der Formel (I) umgesetzt werden.

Verwendet man als Ausgangsstoffe bei der Verfahrensvariante (a) beispielsweise Pentafluorbenzylalkohol und bei Variante (b) 4-Fluor-3-phenoxy-benzaldehyd, so können die Reaktionen bei den beiden Verfahrensvarianten durch folgende Formelschemata skizziert werden :

(a)

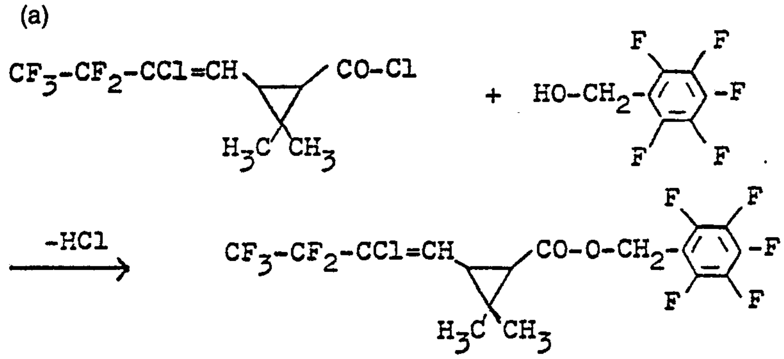


(b)

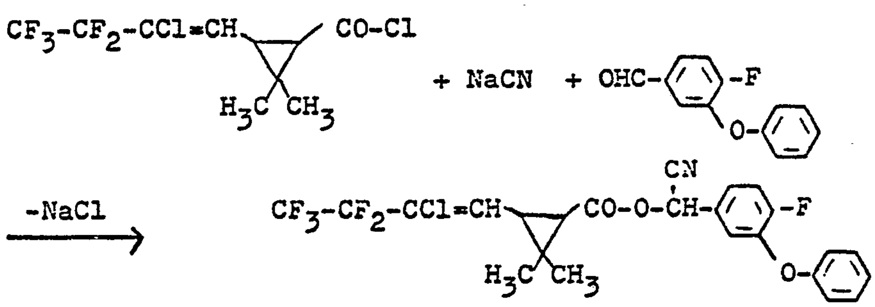


Die als Ausgangsverbindung zu verwendende 3-(2-Chlor-3,3,4,4,4-pentafluor-1-butenyl)-2,2-dimethylcyclopropancarbonsäure ist bereits bekannt (vgl. DE-OS 2 802 962/GB-PS 2 000 764).

Das Säurechlorid der Formel (IIa) erhält man daraus auf übliche Weise, beispielsweise durch Umsetzung mit Thionylchlorid, gegebenenfalls in Gegenwart eines Verdünnungsmittels, wie z. B. Tetrachlorkohlenstoff, bei Temperaturen zwischen 10 und 100 °C.

Die weiter als Ausgangsstoffe zu verwendenden Benzylalkohole sind durch Formel (III) definiert. Vorzugsweise stehen darin $R^1$ und $R^2$ für diejenigen Reste, welche bereits bei der Definition der Reste $R^1$ und $R^2$ in Formel (I) als bevorzugt genannt wurden.

Als Beispiele für die Ausgangsverbindungen der Formel (III) seien genannt :

Pentafluorbenzylalkohol, 4-Fluor-3-phenoxy-benzylalkohol.

Die Ausgangsverbindungen der Formel (III) sind bereits bekannt (vgl. GB-PS 1 078 511, DE-OS 2 621 433, 2 709 264 und 2 739 854).

Die als Ausgangsstoffe verwendbaren Phenoxy-benzaldehyde sind durch Formel (IV) definiert.

Vorzugsweise steht darin $R^2$ für diejenigen Reste, welche bereits bei der Definition von $R^2$ in Formel (I) als bevorzugt genannt wurden.

Als Beispiel sei genannt :

4-Fluor-3-phenoxy-benzaldehyd.

Die Phenoxybenzaldehyde der Formel (IV) sind bereits bekannt (vgl. DE-OS' 2 621 433, 2 709 264 und 2 739 854).

Das Verfahren zur herstellung der neuen Verbindungen der Formel (I) wird in allen Varianten vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran, und Dioxan, Ketone, wie Aceton, Methyl-ethyl-, Methyl-isopropyl- und Methyl-isobutyl-keton, Ester, wie Essigsäure-methylester und -ethylester, Nitrile, wie z. B. Acetonitril und Propionitril, Amide, wie z. B. Dimethylformamid, Dimethylacetamid und N-Methylpyrrolidon, sowie Dimethylsulfoxid, Tetramethylensulfon und Hexamethylphosphorsäuretriamid.

Die Variante (a) des erfindungsgemäßen Verfahrens wird vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Diazabicyclooctan, Diazabicyclononen und Diazabicycloundecen.

Die Variante (b) des erfindungsgemäßen Verfahrens wird in Gegenwart von Wasser und eines der oben genannten organischen Lösungsmittel, soweit mit Wasser nicht mischbar, durchgeführt. Hierfür sind insbesondere die oben genannten Kohlenwasserstoffe geeignet.

Als Katalysatoren werden bei der Verfahrensvariante (b) vorzugsweise Verbindungen verwendet, welche zum Transfer von Anionen aus Wasser in organische Lösungsmittel geeignet sind. Beispiele hierfür sind Benzyl-triethyl-ammonium-hydrogensulfat, Tetrabutylammonium-bromid und Methyl-trioctylammonium-chlorid (Aliquat 336).

In allen Verfahrensvarianten kann die Reaktionstemperatur innerhalb eines größeren Bereichs variiert werden. Im allgemeinen arbeitet man zwischen 0 und 100 °C, vorzugsweise bei 10 bis 50 °C.

Das erfindungsgemäße Verfahren wird im allgemeinen bei Normaldruck durchgeführt.

Zur Durchführung des erfindungsgemäßen Verfahrens werden die Ausgangsstoffe gewöhnlich in angenähert äquimolaren Mengen eingesetzt. Ein Überschuß der einen oder anderen Reaktionskomponente bringt keine wesentlichen Vorteile.

Die Ausgangsstoffe werden in geeigneten Verdünnungsmitteln zusammen gegeben und, gegebenenfalls nach Zugabe eines Säureakzeptors und/oder eines Katalysators, bis zum Reaktionsende gerührt.

Die Aufarbeitung kann nach üblichen Methoden durchgeführt werden, beispielsweise indem man das Reaktionsgemisch gegebenenfalls mit Wasser und/oder einem mit Wasser nicht mischbaren organischen Lösungsmittel, wie z. B. Toluol, verdünnt, die organische Phase abtrennt, mit Wasser wäscht, trocknet, filtriert und vom Filtrat das Lösungsmittel unter vermindertem Druck und bei mäßig erhöhter Temperatur sorgfältig abdestilliert (« andestilliert »).

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis,

Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotaosa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate ; als Dispergiermittel kommen in Frage : z. B. Lignin-Sulfit-ablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und

organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000 000 1 bis zu 100 Gew.-% Wirkstoff, vorzugsweise zwischen 0,000 1 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

Herstellungsbeispiele

Beispiel 1

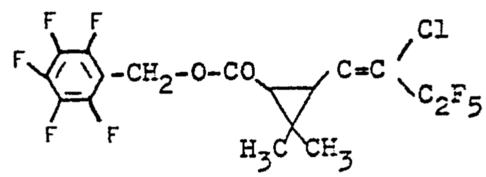

(1)

3,06 g (±)-cis/trans-2,2-Dimethyl-3-(2-chlor-3,3,4,4,4-pentafluor-1-butenyl)-cyclopropan-1-carbonsäurechlorid werden mit 100 ml Toluol und 1,98 g Pentafluor-benzyl-alkohol vermischt. Dann tropft man bei Raumtemperatur unter Rühren 0,72 g Pyridin zu und rührt über Nacht bei Raumtemperatur nach. Dann wird das Reaktionsgemisch mit 150 ml Wasser versetzt, die organische Phase abgetrennt und mit 100 ml Wasser gewaschen. Die organische Phase wird mit Natriumsulfat getrocknet, abfiltriert, einrotiert und destilliert. Man erhält 2,2-Dimethyl-3-(2-chlor-3,3,4,4,4-pentafluor-1-butenyl)-cyclopropan-1-carbonsäurepentafluor-benzylester.

Beispiel 2

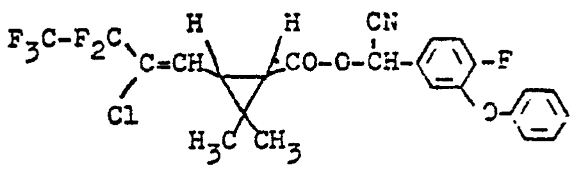

(2)

4,98 g (0,016 Mol) (±)-cis/trans-3-(E/Z-2-Chlor-3,4-pentafluor-but-1-en-1-yl)-2,2-dimethyl-cyclopropancarbonsäurechlorid und 3,46 g (0,016 Mol) 3-Phenoxy-4-fluorbenzaldehyd werden zusammen tropfenweise unter Rühren bei 20-25 °C zu einer Mischung aus 1,26 g Natriumcyanid, 1,9 ml Wasser, 75 ml n-Hexan und 0,4 g Tetrabutylammoniumbromid gegeben. Das Reaktionsgemisch wird dann 4 Stunden bei 20 bis 25 °C gerührt, anschließend mit 150 ml Toluol verdünnt und zweimal mit je 200 ml Wasser gewaschen. Die organische Phase wird über Magnesiumsulfat getrocknet und das Lösungsmittel wird im Wasserstrahlvakuum abdestilliert. Letzte Lösungsmittelreste werden durch kurzes Andestillieren bei 60 °C/l mbar entfernt. Man erhält 5,9 g (71,3 % der Theorie) (±)-cis-trans-3-(E/Z-2-Chlor-3,4-pentafluor-but-1-en-1-yl)-2,2-dimethyl-cyclopropancarbonsäure-(3-phenoxy-4-fluor-α-cyano-benzyl)-ester als gelbes

zähes Öl. Die Struktur wird durch das $^1H$-NMR-Spektrum bewiesen.

$^1H$-NMR-Daten ($CDCl_3$/TMS) τ (ppm) :

Aromatische-H : 2,5-3,12 (m/8 H), benzyl-H : 3,6-3,74 (m/1 H), vinyl-H : 3,19 d und 3,88 d/1 H, cyclopropan-H : 7,38-8,24 (m/2 H) und dimethyl-H : 8,55-8,9 (m/6 H).

Herstellung der Ausgangsverbindung :

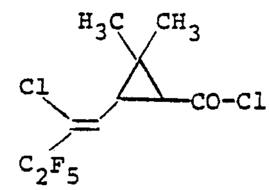

21,5 g (0,07 Mol) (±)-cis/trans-2,2-Dimethyl-3-(2-chlor-3,3,4,4,4-pentafluor-1-butenyl)-cyclopropan-1-carbonsäure werden in 150 ml Tetrachlorkohlenstoff gelöst und 50 ml Thionylchlorid hinzugefügt. Anschließend wird unter Rühren 1 Stunde auf 80 °C erwärmt. Nach Abdestillieren des überschüssigen Thionylchlorid sowie des Lösungsmittels wird der Rückstand im Hochvakuum destilliert. Man erhält 21 g (±) cis/trans-2,2-Dimethyl-3-(2-chlor-3,3,4,4,4-pentafluor-1-butenyl)-cyclopropan-1-carbonsäurechlorid vom Kp = 50-54 °C/0,3 mbar.

## Beispiel A

Drosophila-Test

Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierscheibe (7 cm Durchmesser) aufpipettiert. Man legt diese naß auf die Öffnung eines Glasgefäßes, in dem sich 50 Taufliegen (Drosophila melanogaster) befinden und bedeckt es mit einer Glasplatte.

Nach der gewünschten Zeit bestimmt man die Abtötung in %. Dabei bedeutet 100 %, daß alle Fliegen abgetötet wurden ; 0 % bedeutet, daß keine Fliegen abgetötet wurden.

Bei diesem Test zeigen z. B. die folgenden Verbindungen der Herstellungsbeispiele überlegene Wirksamkeit gegenüber dem Stand der Technik : 1,2

## Beispiel B

Tetranychus-Test (resistent)

Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetätet wurden ; 0 % bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik : 1,2.

## Beispiel C

Grenzkonzentrations-Test/Bodeninsekten

Testinsekt : Phorbia antigua-Maden im Boden.
Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das

Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Valumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungs ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirksamkeit gegenüber dem Stand der Technik : 1,2.

Beispiel D

Grenzkonzentrations-Test/Bodeninsekten

Testinsekt : Tenebrio molitor-Larven im Boden
Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigt z. B. die Verbindung 2 der Herstellungsbeispiele überlegene Wirksamkeit gegenüber dem Stand der Technik.

Beispiel E

Grenzkonzentrations-Test/Bodeninsekten

Testinsekt : Agrotis segetum im Boden
Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebeden Testinsekten in % bestimmt. Der Wirkungsgrad ist 100 %, wen alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik : 2.

Beispiel F

$LT_{100}$-Test

Testtiere : Aedes aegypti
Zahl der Testtiere : 25
Lösungsmittel : Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen,

bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden ; 0 % bedeutet, daß keine Testtiere abgetötet wurden.

Bei diesem Test zeigen z. B. die folgenden Verbindungen der Herstellungsbeispiele überlegene Wirkung gegenüber dem Stand der Technik : 1,2.

Beispiel G

Test mit Boophilus microplus resistent

Lösungsmittel : 35 Gewichtsteile Äthylenglykolmonomethyläther
35 Gewichtsteile Nonylphenolpolylgkyoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach überführung in Plastikbecher und Aufbewahrung in einem klimatisierten Raum wird der Abtötungsgrad bestimmt.

Bei diesem Test zeigen z. B. die folgenden Verbindungen der Herstellungsbeispiele überlegene Wirkung gegenüber dem Stand der Technik : 1,2.

Beispiel H

Test mit Stomoxys calcitrans

Lösungsmittel : 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Stomoxys calcitrans werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z. B. die folgenden verbindungen der Herstellungsbeispiele überlegene Wirkung gegebenüber dem Stand der Technik : 2.

**Ansprüche**

1. 3-(2-Chlor-3,3,4,4,4-pentafluor-1-butenyl)-2,2-dimethyl-cyclopropancarbonsäureester der Formel I

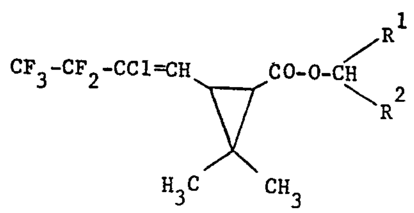

(I)

in welcher

$R^1$ für Wasserstoff oder Cyano steht und

$R^2$ für einen durch Fluor und/oder Phenoxy substituierten Phenyl-Rest steht, mit der Maßgabe, daß der Rest $R^2$ insgesamt wenigstens einen Fluor-Substituenten enthält.

2. Verbindungen gemäß Anspruch 1, wobei

$R^1$ für Wasserstoff oder Cyano steht und

$R^2$ für Pentafluorphenyl oder 4-Fluor-3-phenoxy-phenyl steht.

3. Verfahren zur Herstellung der Verbindungen der Formel I

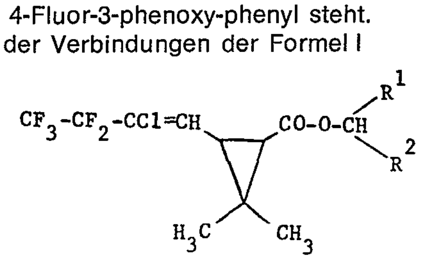

(I)

in welcher

$R^1$ für Wasserstoff oder Cyano und

$R^2$ für einen durch Fluor und/oder Phenoxy substituierten Phenyl-Rest steht, mit der Maßgabe, daß der Rest $R^2$ insgesamt wenigstens einen Fluor-Substituenten enthält, dadurch gekennzeichnet, daß man 3-(2-Chlor-3,3,4,4-pentafluor-1-butenyl)-2,2-dimethyl-cyclopropancarbonsäure der Formel II

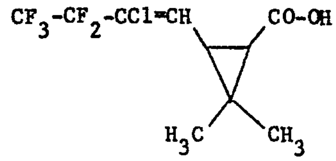

(II)

oder reaktionsfähige Derivate derselben, mit Benzylalkoholen der Formel III

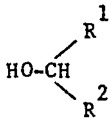

(III)

in welcher

$R^1$ und $R^2$ die oben angebene Bedeutung haben, oder mit reaktionsfähigen Derivaten derselben, gegebenenfalls in Gegenwart von Säureakzeptoren und/oder Katalysatoren und gegebenenfalls unter Verwendung von Verdünnungsmitteln umsetzt.

4. Schädlingsbekämpfungsmittel, gekennzeichnet durch einen Gehalt an mindestens einem 3-(2-Chlor-3,3,4,4,4-pentafluor-1-butenyl)-2,2-dimethyl-cyclopropan-carbonsäureester der Formel (I).

5. Verwendung von 3-(2-Chlor-3,3,4,4,4-pentafluor-1-butenyl)-2,2-dimethyl-cyclopropancarbonsäureestern der Formel (I) zur Bekämpfung von Schädligen.

6. Verfahren zur Herstellung von Schädlingsbekämpfungsmitteln, dadurch gekennzeixhnet, daß man 3-(2-Chlor-3,3,4,4,4-pentafluor-1-butenyl)-2,2-dimethyl-cyclopropancarbonsäureester der Formel (I) mit Streckmitteln und/oder oberflächenaktiven Mitteln vermischt.

**Claims**

1. 3-(2-chloro-3,3,4,4,4-pentafluoro-1-butenyl)-2,2-dimethylcyclopropanecarboxylic acid esters of the formula I

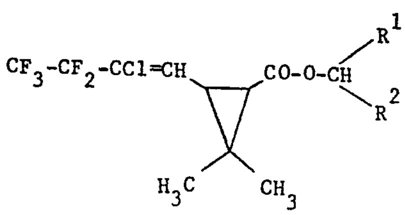

(I)

in which

$R^1$ represents hydrogen or cyano and

$R^2$ represents a phenyl radical which is substituted by fluorine and/or phenoxy, with the proviso that the radical $R^2$ contains a total of at least one fluorine-substituent.

2. Compounds according to Claim 1, in which

$R^1$ represents hydrogen or cyano and

$R^2$ represents pentafluorophenyl or 4-fluoro-3-phenoxyphenyl.

3. Process for the preparation of compounds of the formula I

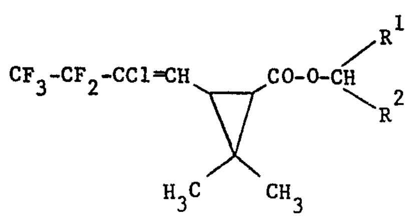

(I)

in which

$R^1$ represents hydrogen or cyano and

$R^2$ represents a phenyl radical which is substituted by fluorine and/or phenoxy, with the proviso that the radical $R^2$ contains a total of at least one fluorine-substituent, characterised in that 3-(2-chloro-3,3,4,4-pentafluoro-1-butenyl)-2,2-dimethyl-cyclopropanecarboxylic acid of the formula II

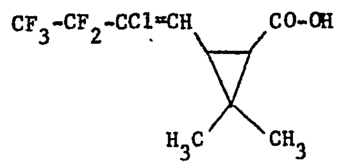

(II)

or reactive derivatives thereof, is reacted with benzyl alcohols of the formula III

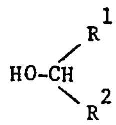

(III)

in which

$R^1$ and $R^2$ have the meaning indicated above, ot with reactive derivatives thereof, if appropriate in the presence of acid acceptors and/or catalysts and if appropriate using diluents.

4. Agents for combating pests, characterised in that they contain at least one 3-(2-chloro-3,3,4,4,4-pentafluoro-1-butenyl)-2,2-dimethyl-cyclopropane-carboxylic acid ester of the formula (I).

5. Use of 3-(2-chloro-3,3,4,4,4-pentafluoro-1-butenyl)-2,2-dimethyl-cyclopropanecarboxylic acid esters of the formula (I) for combating pests.

6. Process for the preparation of agents for combating pests, characterised in that 3-(2-chloro-3,3,4,4,4-pentafluoro-1-butenyl)-2,2-dimethyl-cyclopropanecarboxylic acid esters of the formula (I) are mixed with extenders and/or surface-active agents.

**Revendications**

1. Esters de l'acide 3-(2-chloro-3,3,4,4,4-pentafluoro-1-butényl)-2,2-diméthylcyclopropane-carboxylique de formule I

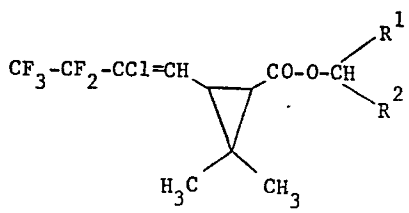

(I)

dans laquelle

$R^1$ représente l'hydrogène ou un groupe cyano, et

$R^2$ représente un reste phényle substitué par le fluor et/ou un groupe phénoxy, étant spécifié que le reste $R^2$ contient au total au moins un substituant fluor.

2. Composés selon la revendication 1, dans lesquels

$R^1$ représente l'hydrogène ou le groupe cyano, et

$R^2$ représente un groupe pentafluorophényle ou 4-fluoro-3-phénoxyphényle.

3. Procédé de préparation des composés de formule I

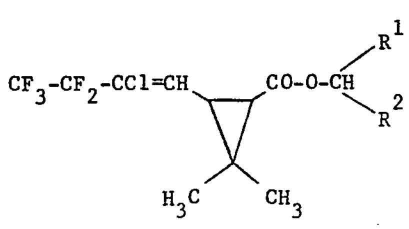

(I)

dans laquelle

$R^1$ représente l'hydrogène ou le groupe cyano, et

$R^2$ représente un reste phényle substitué par le fluor et/ou un groupe phénoxy, étant spécifié que le reste $R^2$ contient au total au moins un substituant fluor, caractérisé en ce que l'on fait réagir l'acide 3-(2-chloro-3,3,4,4,4-pentafluoro-1-buténgl)-2,2-diméthylcyclopropane-carboxylique de formule II

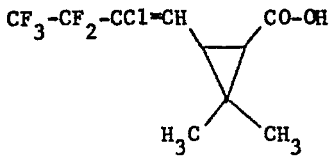

(II)

ou des dérivés réactifs de cet acide, avec des alcools benzyliques de formule III

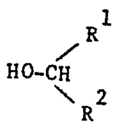

(III)

dans laquelle

$R^1$ et $R^2$ ont les significations indiquées ci-dessus, ou avec des dérivés réactifs de ces alcools, éventuellement en présence d'accepteurs d'acides et/ou de catalyseurs et le cas échéant en utilisant des diluants.

4. Produit pesticide caractérisé en ce qu'il contient au moins un ester de l'acide 3-(2-chloro-3,3,4,4,4-pentafluoro-1-buténgl)-2,2-diméthylcyclopropane-carboxylique de formule I.

5. Utilisation des esters de l'acide 3-(2-chloro-3,3,4,4,4-pentafluoro-1-butényl)-2,2-diméthylcyclopropane-carboxylique de formule I dans la lutte contre les parasites.

6. Procédé de préparation de produits pesticides caractérisé en ce que l'on mélange des esters de l'acide 3-(2-chloro-3,3,4,4,4-pentafluoro-1-butényl)-2,2-diméthylcyclopropane-carboxylique de formule I avec des diluants et/ou des agents tensioactifs.